# EUROPÄISCHE PATENTSCHRIFT

(11) **EP 0 659 744 B1**
(45) Veröffentlichungstag und Bekanntmachung des Hinweises auf die Patenterteilung: **28.02.2001**
(21) Anmeldenummer: 94120044.6
(22) Anmeldetag: 17.12.1994
(51) Int. Cl.: C07D 223/08, C07D 281/02, C07D 267/04

(54) **Verbessertes Verfahren zur Herstellung von Hexahydroazepinonen und Hexahydroazepinolen**
Process of preparation of hexahydroazepinones and hexahydroazepinoles
Procédé pour la préparation des hexahydroazépinones et hexahydroazépinoles

(30) Priorität: 18.12.1993 DE 4343409
(43) Veröffentlichungstag der Anmeldung: 28.06.1995
(73) Patentinhaber: ASTA Medica Aktiengesellschaft, 01277 Dresden (DE)
(72) Erfinder: Olbrich, Dr. Alfred, D-63179 Obertshausen (DE); Engel, Prof. Jürgen, D-93755 Alzenau (DE); Kutscher, Dr. Bernd, D-93477 Maintal (DE); Möller, Roland, D-63546 Hammersbach (DE)
(74) Vertreter: Decker, Karl-Ludwig

(56) Entgegenhaltungen:
- DE-A- 3 530 793
- DE-C- 2 206 385
- US-A- 3 813 384
- COLLECTION OF CZECHOSLOVAK CHEMICAL COMMUNICATIONS, Bd.51, Nr.9, 1986, PRAGUE Seiten 2034 - 2048 ZDENEK POLIVKA ET AL. 'Heterocyclic ethers derived from 6,11-dihydrodibenzo[b,c]thiepin-11-ols and ............'
- BULLETIN OF THE CHEMICAL SOCIETY OF JAPAN, Bd.31, Nr.4, Mai 1958, JAPAN Seiten 418 - 422 SHIRO MOROSAWA 'Studies on Seven-membered Heterocyclic compounds containing Nitrogen. II....'
- ORGANIC REACTIONS, Bd.15, 1967, NEW YORK Seiten 1 - 203 JOHN P. SCHAEFFER 'The Dieckmann condensation'

## Beschreibung

Die vorliegende Erfindung betrifft ein Verfahren zur Herstellung von Hexahydroazepinonen und Hexahydroazepinolen der allgemeinen Formel I 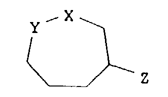 in welcher
X = CH₂
Y = N-R, N-CH₃ oder = N-CH₂-CH₂-C₆H₅ und
Z = O, -OH bedeutet wobei in jedem Fall gilt R = (C₁-C₅)-Alkyl, (C₁-C₅)-Alkylphenyl sowie deren Säureadditionssalze.

Heterocyclische Siebenringketone stellen wichtige Zwischenprodukte dar für die Synthese pharmakologisch aktiver Prinzipien. Hierzu zählen antiasthmatisch und antiallergisch wirksame Strukturen vom Typ des Azelastins (INN) und des Flezelastins (INN) (z.B. DE-OS 3634942 / Drugs Of The Future 12 (3), 283 (1987 / EP 488209), aber auch Strukturen zur Behandlung von Krankheiten des Zentralnervensystems, wie Parkinsonismus, Hyperprolactinämie und Schizophrenie oder zur Behandlung cardiovaskulärer Erkrankungen (z. B. DE-OS 3820775). Auch fungizid wirksame Substanzen mit heterocyclischer Siebenring-Teilstruktur sind bekannt.
Darüberhinaus zeigen Derivate vom Phospholipid-Typ mit heterocyclischer Siebenring-Teilstruktur in der Kopfkomponente antineoplastische Aktivität.

Auffällig sind auch die stark variierenden Ausbeuten, sogar bei der Herstellung identischer Produkte durch unterschiedliche Arbeitsgruppen.
Alternative Herstellverfahren wie
- Friedel-Crafts-Acylierung (J. Chem. Soc., Perkin Trans. I 1992, 445)
- Ringerweiterung mit Diazomethan (Bull. Chem. Soc., Japan, 31, 418 (1958); Coll. Czech. Chem. Commun. 51, 2034 (1986))
- Ozonolyse von Cyclohexenon gefolgt von einer reduktiven Aminocyclisierung ( Synth. Commun. 21(7), 881 (1991))
führten nicht zu befriedigenden Ausbeuten an den gewünschten Produkten, abgesehen von der technischen Realisierung der Ozonolyse bzw. der Umgang mit Diazomethan.

In der DE A 220 6385 ist ein Verfahren zur Herstellung von N-Benzylhexahydroazepinonen mittels Dickmann Kondesation beschrieben unter Verwendung von Kalium tertiär Butylat in Toluol und einer Konzentration von 0,6 mol/l des Dicarbonsäureesters. Dieses Verfahren erzielt jedoch nur eine Ausbeute von 63 % und verwendet 40% Basenüberschuß, was sich stark umweltbelastend auswirkt.

Aufgabe der Erfindung ist die Ausarbeitung eines Verfahrens zur Herstellung von heterocyclischen Siebenringketonen in technischen Dimensionen basierend auf der bekannten Dieckmann-Kondensations-Reaktion.

Die Erfindung betrifft die Herstellung von Salzen heterocyclischer Siebenringketone mittels Dieckmann-Kondensation.
Hierbei wird so vorgegangen, daß ein in bekannter Weise zugänglicher 1,8-Dicarbonsäureester in inertem Lösungsmittel mit starken Basen umgesetzt wird. Nach saurer Hydrolyse des entstehenden cyclischen β-Enolat-Carbonsäureesters wird der Ester ohne Isolierung verseift und thermisch decarboxyliert. Das gebildete heterocyclische Siebenringketon wird anschließend in Form seines Säureadditionssalzes aus dem Reaktionsgemisch abgetrennt und in reiner Form isoliert.

Überraschenderweise wurde gefunden, daß die Parameter lange Reaktionszeit, hohe Basenüberschüsse und starke Verdünnung des 1,8-Dicarbonsäureesters im verwendeten wasserfreien Lösungsmittel nicht in besonderer Weise die Höhe der Ausbeute beeinflussen. Bei Reaktionszeiten von 1-6, vorzugsweise 1-3 Stunden und Basenüberschüssen von 0 bis maximal 20% konnten technische Bedingungen gefunden werden, Verdünnung zu realisieren, ohne auf große wasserfreie Lösungsmittelmengen zurückzugreifen und somit die Raum-Zeit-Ausbeute erheblich zu steigern.

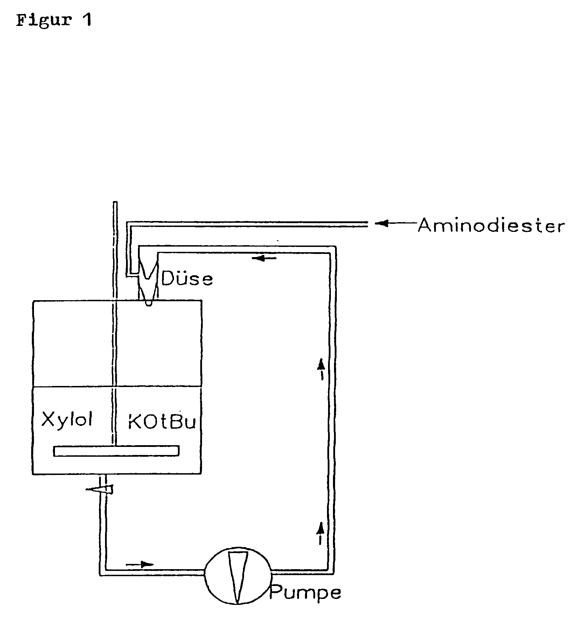

Hierzu wurden zwei Methoden entwickelt.
1. Die in inertem Lösungsmittel gelöste Base wird im Kreislauf geführt. In diesem Kreislauf befindet sich eine Strahldüse ähnlich dem Prinzip der Wasserstrahlpumpe. In den Seiteneinlaß der Düse wird der Dicarbonsäureester unverdünnt eindosiert (siehe Figur 1).
2. Der aufsteigende Dampf längs einer gepackten Füllkörperkolonne wird zur Verdünnung des in unverdünnter Form zudosierten Dicarbonsäureesters, der am Kopf dieser Kolonne dosiert wird, genutzt. Als praktischer Nebeneffekt wird der Diester hierbei bereits auf Reaktionstemperatur gebracht und kann bei Verwendung eines geeigneten Lösungsmittels auch längs der Kolonne absolutiert, bzw. von leichtflüchtigen Nebenprodukten, wie Restlösungsmitteln, befreit werden. Die Konzentration bei den beiden voranstehend beschriebenen Methoden bewegen sich zwischen 0,1 bis 1,5 vorzugsweise zwischen 0,3 bis 0,9 mol/l (Dicarbonsäureester zu anfänglich eingesetzter Lösungsmittelmenge).
   Gleichzeitig zur Zugabe des Dicarbonsäureesters wird ein azeotropes Lösungsmittelgemisch am Kopf der Kolonne abgetrennt, und zwar soviel wie der 1-3fachen Volumenmenge an zudosiertem Dicarbonsäureester entspricht.

Als Lösungsmittel kommen in Betracht:
aliphatische Ether wie beispielsweise
- Diethylether
- Tetrahydrofuran
- Dioxan
aromatische Kohlenwasserstoffe wie beispielsweise
- Benzol
- Toluol
- o-Xylol
- m-Xylol
- p-Xylol
- Xylol (Isomerengemisch)
- Mesitylen
als Basen können:
- Alkali- und Erdalkali-Hydride
- Alkali- und Erdalkali-Amide
- Alkali- und Erdalkali-Alkoholate
eingesetzt werden.

Als Alkoholkomponenten für die 1,8-Dicarbonsäureester kommen in Frage
- Alkylalkohole
- Isoalkylalkohole
- Cycloalkylalkohole
sowie Aralkyl- oder Arylalkohole

Die vorliegende Erfindung wird durch nachfolgende Beispiele näher erläutert.

### Beispiel 1

Zu einer im Kreislauf gepumpten Lösung von 123,4 g Kalium-tert.-butylat in 1,3 l siedendem Xylol werden unter Stickstoff im Seitenstrom 245,3 g 4-(2-Carbethoxyethyl-methyl-amino)buttersäure-ethylester binnen 2 Stunden gleichmäßig zudosiert und weitere 0,5 Stunden nachreagieren lassen. Hierbei werden 750 ml Destillat abgenommen. Das Reaktionsgemisch wird bei 50-80°C durch rasche Zugabe in ein Gemisch aus 300 ml konzentrierter (37%iger) Salzsäure und 500 g gestückeltem Eis hydrolysiert. Die abgetrennte organische Phase wird noch zweimal mit je 150 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 700 ml Isopropanol gelöst, heiß vom ungelösten Kaliumchlorid abfiltriert und bei 0 bis -5°C kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 141,6 g (86,6%) 1-Methyl-perhydroazepin-4-on-HCl.
Schmelzpunkt: 167°C (Zers.)

### Beispiel 2

Zu einer im Kreislauf gepumpten Lösung von 123,4 g Kalium-tert.-butylat in 1,3 l siedendem Xylol werden unter Stickstoff im Seitenstrom 245,3 g 4-(2-Carbethoxyethyl-methyl-amino)buttersäure-ethylester gelöst in 900 ml Xylol binnen 3 Stunden gleichmäßig zudosiert und weitere 1,5 Stunden nachreagieren lassen. Hierbei werden 1500 ml Destillat abgenommen. Das Reaktionsgemisch wird bei 50-80°C durch rasche Zugabe in ein Gemisch aus 300 ml konzentrierter (37%iger) Salzsäure und 500 g gestückeltem Eis hydrolysiert. Die abgetrennte organische Phase wird noch zweimal mit je 150 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 700 ml Isopropanol gelöst, heiß vom ungelösten Kaliumchlorid abfiltriert und bei 0 bis -5°C kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 145,9 g (89,2%) 1-Methyl-perhydroazepin-4-on-HCl.
Schmelzpunkt: 163-164°C (Zers.)

### Beispiel 3

Zu einer im Kreislauf gepumpten Lösung von 43,6 g Kalium-tert.-butylat in 1,65 l siedendem Xylol werden unter Stickstoff im Seitenstrom 100 g 4-(2-Carbethoxyethyl-benzyl-amino)buttersäure-ethylester gelöst in 780 ml Xylol binnen 3 Stunden gleichmäßig zudosiert und weitere 1,5 Stunden nachreagieren lassen. Hierbei werden 800 ml Destillat abgenommen. Das Reaktionsgemisch wird bei 50-80°C durch rasche Zugabe in ein Gemisch aus 200 ml konzentrierter (37%iger) Salzsäure und 500 g gestückeltem Eis hydrolysiert. Die abgetrennte organische Phase wird noch zweimal mit je 100 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird aus Isopropanol erschöpfend extrahiert und kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 60,3 g (80,8%) 1-Benzyl-perhydroazepin-4-on-HCl.
Schmelzpunkt: 191-193°C (Zers.)

### Beispiel 4

Zu einer Lösung von 50,0 kg Kalium-tert.-butylat in 400 l siedendem Xylol werden unter Stickstoff am Kolonnenkopf 90,0 kg 4-(2-Carbethoxyethyl-methyl-amino)buttersäure-ethylester binnen 2-3 Stunden gleichmäßig zudosiert und weitere 1-1,5 Stunden nachreagieren lassen. Hierbei werden ca. 160 l Destillat abgenommen. Das Reaktionsgemisch wird bei 50-80°C durch rasche Zugabe in ein Gemisch aus 80 l konzentrierter (37%iger) Salzsäure und 100 kg gestückeltem Eis hydrolysiert. Die abgetrennte organische Phase wird noch zweimal mit je 50 l halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 400 l Isopropanol gelöst, heiß vom ungelösten Kaliumchlorid abfiltriert und bei 0 - -5°C kristallisiert. Der abfiltrierte Rückstand wird bei erhöhter Temperatur im Vakuum bis zur Gewichtskonstanz getrocknet. Man erhält 43,2 kg (72,0%) 1-Methyl-perhydroazepin-4-on-HCl.
Schmelzpunkt: 162-165°C (Zers.)

### Beispiel 5

Zu einer Lösung von 61,7 g Kalium-tert.-butylat in 1,5 l siedendem Xylol werden unter Einleiten von Stickstoff am Kopf einer Füllkörperkolonne 168,8 g 4-(2-Carbethoxyethyl-(2-phenylethyl)-amino)buttersäure-ethylester (Gehalt: 94%) binnen 2-3 Stunden gleichmäßig zudosiert und weitere 1-1,5 Stunden nachreagieren lassen. Hierbei werden ca. 500 ml Destillat abgenommen. Das Reaktionsgemisch wird bei 50-80°C durch rasche Zugabe in ein Gemisch aus 200 ml konzentrierter (37%iger) Salzsäure und 300 g gestückeltem Eis hydrolysiert. Die abgetrennte organische Phase wird noch zweimal mit je 100 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 250 ml Isopropanol heiß suspendiert und in der Kälte kristallisiert. Das Kristallisat wird aus Isopropanol erschöpfend extrahiert und kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 107,7 g (89,7%) 1-(2-Phenylethyl)-perhydroazepin-4-on-HCl.
Schmelzpunkt: 196-198°C (Zers.)

### Beispiel 6

Zu einer Lösung von 30,0 kg Kalium-tert.-butylat in 400 l siedendem Xylol werden unter Stickstoff am Kopf einer Füllkörperkolonne 84,5 kg 4-(2-Carbethoxyethyl-(2-phenylethyl)-amino)buttersäure-ethylester(Gehalt: 92,2%) binnen 2-3 Stunden gleichmäßig zudosiert und weitere 1-1,5 Stunden nachreagieren lassen. Hierbei werden ca. 150 l Destillat abgenommen. Das Reaktionsgemisch wird bei 50-80°C durch rasche Zugabe in ein Gemisch aus 60 l konzentrierter (37%iger) Salzsäure und 60 kg gestückeltem Eis hydrolysiert. Die abgetrennte organische Phase wird noch zweimal mit je 30 l halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird aus Isopropanol erschöpfend extrahiert und kristallisiert. Der abfiltrierte Rückstand wird bei erhöhter Temperatur im Vakuum bis zur Gewichts-konstanz getrocknet. Man erhält 40,0 kg (67,8%) 1-(2-Phenylethyl)-perhydroazepin-4-on-HCl.
Schmelzpunkt: 196-198°C (Zers.)
Elementaranalyse: ber.: C66,26 H7,95 N5,52 gef.: C66,24 H7,94 N5,43 Gehalt (Cl-): 100,28%

### Beispiel 7

Zu einer Lösung von 18,9 g Natriumboranat in 100 ml Wasser setzt man 100 ml 1 N Natronlauge zu. Bei einer Innentemperatur von 0 - 5°C tropft man eine Lösung von 163,6 g 1-Methyl-perhydroazepin-4-on-HCl in 100 ml Wasser zu. Man rührt 2 Stunden bei 0 - 5°C und 2 Stunden bei Raumtemperatur nach. Durch Zugabe von halb-konzentrierter Salzsäure wird der pH-Wert auf 2-3 eingestellt. Man engt im Vakuum zur Trockene ein, nimmt den Rückstand in 600 ml Isopropanol auf, trennt bei 60 - 75°C die anorganischen Salze ab und kristallisiert das Produkt in der Kälte aus. Das Produkt wird abgesaugt und im Vakuum bei erhöhter Temperatur bis zur Gewichtskonstanz ge-trocknet. Man erhält 149 g (90%) 1-Methyl-perhydroazepin-4-ol-HCl.
Schmelzpunkt: 156-158°C.

### Beispiel 8

Zu einer Suspension von 5,0 kg Natriumhydrid (80%ig in Weißöl) in 200 l siedendem Xylol werden unter Stickstoff 34,0 kg 4-(2-Carbethoxy-ethyl-methyl-amino)buttersäure-ethylester gelöst in 400 l Xylol binnen 2-3 Stunden gleichmäßig zudosiert und weitere 0,5 Stunden nachreagieren lassen. Hierbei werden ca. 400 l Destillat abgenommen. Das Reaktionsgemisch wird bei 50-80°C durch rasche Zugabe in ein Gemisch aus 35 l konzentrierter (37%iger) Salzsäure und 60 kg gestückeltem Eis hydrolysiert. Die abgetrennte organische Phase wird noch zweimal mit je 30 l halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 100 l Isopropanol gelöst, heiß vom ungelösten Kaliumchlorid abfiltriert und bei 0 bis -5°C kristallisiert. Der abfiltrierte Rückstand wird bei erhöhter Temperatur im Vakuum bis zur Gewichtskonstanz getrocknet. Man erhält 11,7 kg (52,0%) 1-Methyl-perhydro-azepin-4-on-HCl.
Schmelzpunkt: 162-164°C.

### Beispiel 9

Zu einer Lösung von 39,0 g Natriumhydrid (80%ig in Weißöl) und 75 ml Ethanol in 1,3 l siedendem Xylol werden unter Einleiten von Stickstoff 245,3 g 4-(2-Carbethoxyethyl-methylamino)buttersäure-ethylester binnen 1,75 Stunden gleichmäßig zudosiert und weitere 0,5 Stunden nachreagieren lassen. Hierbei werden ca. 700 ml Destillat abgenommen. Das Reaktionsgemisch wird bei 80-100°C durch rasche Zugabe in ein Gemisch aus 300 ml konzentrierter (37%iger) Salzsäure und 500 g gestückeltem Eis hydrolysiert. Die abgetrennte organische Phase wird noch zweimal mit je 150 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird aus 600 ml Isopropanol heiß extrahiert und kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 44,3 g (27,1%) 1-Methyl-perhydroazepin-4-on-HCl.
Schmelzpunkt: 159-161°C (Zers.)

### Beispiel 10

Zu einer Lösung von 61,7 g Kalium-tert.-butylat in 1,5 l siedendem Xylol werden unter Einleiten von Stickstoff am Kopf einer Füllkörperkolonne 181,8 g 4-(2-Carbethoxyethyl-(2-phenylethyl)-amino)buttersäure-n-butylester (Gehalt: 93%) binnen 2-3 Stunden gleichmäßig zudosiert und weitere 1-1,5 Stunden nachreagieren lassen. Hierbei werden ca. 500 ml Destillat abgenommen. Das Reaktionsgemisch wird bei 50-80°C durch rasche Zugabe in ein Gemisch aus 200 ml konzentrierter (37%iger) Salzsäure und 300 g gestückeltem Eis hydrolysiert. Die abgetrennte organische Phase wird noch zweimal mit je 100 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird in 250 ml Isopropanol heiß suspendiert und in der Kälte kristallisiert. Das Kristallisat wird aus Isopropanol erschöpfend extrahiert und kristallisiert. Der abfiltrierte Rückstand wird bei erhöhter Temperatur im Vakuum bis zur Gewichtskonstanz getrocknet. Man erhält 95,4 g (80,8%) 1-(2-Phenylethyl)-perhydroazepin-4-on-HCl.
Schmelzpunkt: 196-197°C (Zers.)

### Beispiel 11

Zu einer Lösung von 61,7 g Kalium-tert.-butylat in 1,5 l siedendem Xylol werden unter Einleiten von Stickstoff am Kopf einer Füllkörperkolonne 153,7 g 4-(2-Carbometh-oxyethyl-(2-phenylethyl)-amino)buttersäure-methylester (Gehalt: 96%) binnen 2-3 Stunden gleichmäßig zudosiert, wobei sich ein schwer rührbarer Niederschlag bildet, der sich bei der Nachreaktionszeit vollständig auflöst und weitere 1-1,5 Stunden nachreagieren lassen. Hierbei werden ca. 500 ml Destillat abgenommen. Das Reaktionsgemisch wird bei 50-80°C durch rasche Zugabe in ein Gemisch aus 200 ml konzentrierter (37%iger) Salzsäure und 300 g gestückeltem Eis hydrolysiert. Die abgetrennte organische Phase wird noch zweimal mit je 100 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird aus Isopropanol erschöpfend extrahiert und kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 105,4 g (86,5%) 1-(2-Phenylethyl)-perhydroazepin-4-on-HCl.
Schmelzpunkt: 196-198°C (Zers.)

### Beispiel 12

Zu einer Lösung von 123,4 g Kalium-tert.-butylat in 1,3 l siedendem Xylol werden unter Einleiten von Stickstoff 245,3 g 4-(2-Carbethoxyethyl-methylamino)buttersäure-ethylester binnen 3 Minuten zugesetzt, wobei rasch 300 ml Lösungsmittel abdestillieren, und weitere 0,5 Stunden nachreagieren lassen. Hierbei werden weitere 350 ml Destillat abgenommen. Das Reaktionsgemisch wird bei 80-100°C durch rasche Zugabe in ein Gemisch aus 300 ml konzentrierter (37%iger) Salzsäure und 500 g gestückeltem Eis hydrolysiert. Die abgetrennte organische Phase wird noch zweimal mit je 150 ml halbkonzentrierter Salzsäure nachgewaschen. Die vereinten wäßrigen Extrakte werden 2 Stunden zum kräftigen Rückfluß erhitzt und anschließend im Vakuum zur Trockene eingeengt. Der Rückstand wird aus 600 ml Isopropanol heiß extrahiert und kristallisiert. Der abfiltrierte Rückstand wird bei erhöhter Temperatur im Vakuum bis zur Gewichtskonstanz getrocknet. Durch Einengen der Mutterlauge auf ein Volumen von ca. 100 ml läßt sich eine Zweitfraktion gewinnen. Man erhält 78,3 g (47,9%) 1-Methyl-perhydroazepin-4-on-HCl.
Schmelzpunkt: 164-165°C (Zers.)

## Patentansprüche

1. Verfahren zur Herstellung von Hexahydroazepinonen der allgemeinen Formel I 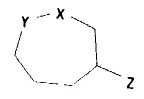 in welcher
X = CH₂
Y = N-R, N-CH₃ oder = N-CH₂-CH₂-C₆H₅ und
Z = O bedeutet
wobei in jedem Fall gilt R = (C₁-C₅)-Alkyl, (C₁-C₅)-Alkylphenyl
sowie deren Salze mittels Dieckmann-Kondensation unter Verwendung starker Basen in inerten Lösungsmitteln gekennzeichnet durch folgende Merkmale:
a) daß man einen Überschuß bis zu maximal 20 % an starken Basen verwendet
b) daß man die Kondensationsreaktion bei Reaktionszeiten von 1 bis 6, vorzugsweise 1 bis 3 Stunden durchführt
c) daß man mit Konzentrationen von 0,1 bis 1,5 vorzugsweise 0,3 bis 0,9 mol/l des zu kondensierenden , unverdünnt zulaufenden, Dicarbonsäureesters pro Liter Lösungsmittel arbeitet
d) daß man unter Vermeidung der üblichen Verdünnungstechnik entweder
die Reaktion in einer geschlossenen Vorrichtung durchführt, wobei die gelöste Base im Kreislauf geführt und der unverdünnte Dicarbonsäureester in diesen Kreislauf unter Verwendung einer Strahldüse zudosiert wird oder
daß das aufsteigende, entstandene Kondensat gleichzeitig in einer Füllkörperkolonne zur Verdünnung und Erwärmung des unverdünnt am Kopf der Kolonne zudosierten Dicarbonsäureesters dient

2. Verfahren zur Herstellung und Isolierung von Hexahydrazepinolen sowie deren Salze durch Reduktion der nach Anspruch 1 hergestellten Hexahydroazepinone mittels komplexer Hydride oder Wasserstoff am hydrogenolytisch aktiven Kontakt in Wasser oder Alkoholen.

3. Verfahren zur Herstellung von Azelastin-Hydrochlorid dadurch gekennzeichnet, daß man die gemäß Anspruch 1 hergestellte Verbindung I, worin X= CH₂, Y=N-CH₃ und Z= O bedeutet, in Form ihres Säureadditionssalzes mit Salzsäure
a) in organischer Lösung umsetzt mit Benzoylhydrazin und die Zwischenverbindung ohne Isolierung zu einem Benzoyl-azepinyl-hydrazin reduziert und
b) diese Verbindung nach saurer Abspaltung des Benzoylrestes in Wasser oder organischem Lösungsmittel mit 2-(4-Chlorphenacetyl)benzoesäure in Azelastin überführt und in Form eines Säureadditionssalzes mit Säure isoliert.

4. Verfahren zur Herstellung von Flezelastin-Hydrochlorid dadurch gekennzeichnet, daß man die nach Anspruch 1 hesrgestellte Verbindung I worin X=CH₂, Y=N-CH₂-CH₂ C₆H₅ und Z=O bedeutet, in Form ihres Säureaddionssalzes mit Säure
a) in organischer Lösung umsetzt mit Benzoylhydrazin und die Zwischenverbindung ohne Isolierung zu einem Benzoyl-azepinyl-hydrazin reduziert.
b)diese Verbindung nach saurer Abspaltung des Benzoylrestes in Wasser oder organischen Lösungsmitteln mit 2-(4-Fluorphenacetyl)benzoesäure in Flezelastin überführt und in Form eines Säureadditionssalzes mit Säure isoliert.

## Claims

1. Method for the preparation of hexahydroazepinones and hexahydroazepinoles of the general formula I 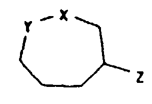 wherein
X denotes CH₂
Y denotes N-R, N-CH₃ or N-CH₂-CH₂-C₆H₅ and
Z denotes O,
wherein in each case R represents (C₁-C₅)-alkyl, (C₁-C₅)-alkylphenyl and salts thereof, by means of the Dieckmann condensation using strong bases in inert solvents, characterised by the following features:
a) that an excess of up to a maximum of 20% of strong bases is used
b) that the condensation reaction is carried out over reaction times of from 1 to 6 hours, preferably from 1 to 3 hours
c) that the operation is conducted using concentrations of from 0.1 to 1.5 mol, preferably 0.3 to 0.9 mol, of the undiluted dicarboxylic ester flowing in to be condensed, per litre of solvent
d) that, avoiding the conventional dilution technique, either
the reaction is carried out in a closed device, with the dissolved base being circulated and the undiluted dicarboxylic ester being charged into the circulation system by means of a jet nozzle, or
that the ascending condensate formed is used simultaneously in a packed column for the dilution and heating of the undiluted dicarboxylic ester introduced at the top of the column.

2. Method for the preparation and isolation of hexahydroazepinoles and salts thereof through reduction of the hexahydroazepinones prepared according to claim 1 by means of complex hydrides or hydrogen on a hydrogenolytically active contact in water or alcohols.

3. Method for the preparation of azelastine hydrochloride, characterised in that the compound I prepared according to claim 1, wherein X denotes CH₂, Y denotes N-CH₃ and Z denotes O, in the form of its acid addition salt with hydrochloric acid
a) is reacted in organic solution with benzoylhydrazine and the intermediate compound is reduced, without being isolated, to form a benzoylazepine hydrazine and
b) the latter compound, following acidic splitting-off of the benzoyl radical in water or organic solvent, by means of 2-(4-chlorophenacetyl)-benzoic acid is converted into azelastine and isolated in the form of an acid addition salt by means of acid.

4. Method for the preparation of flezelastine hydrochloride, characterised in that the compound I prepared according to claim 1, wherein X denotes CH₂, Y denotes N-CH₂CH₂-C₆H₅ and Z denotes O, in the form of its acid addition salt with acid
a) is reacted in organic solution with benzoylhydrazine and the intermediate compound is reduced, without being isolated, to form a benzoylazepine hydrazine
b) the latter compound, following acidic splitting-off of the benzoyl radical in water or organic solvents, by means of 2-(4-fluorophenacetyl)-benzoic acid is converted into flezelastine and isolated in the form of an acid addition salt by means of acid.

## Revendications

1. Procédé de préparation des hexahydroazépinones et hexahydroazépinoles de formule générale I, 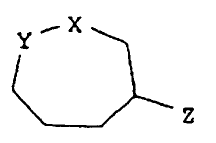 dans laquelle
X signifie CH₂
Y = N-R, N-CH₃ ou = N-CH₂-CH₂-C₆H₅ et
Z = O, -OH, pour lesquels dans chaque cas R est égal à un (C₁-C₅)-alkyle, un (alkyle en C₁-C₅)phényle ainsi que leurs sels
au moyen d'une condensation de Dieckmann en utilisant des bases fortes dans des solvants inertes,
caractérisé par
les paramètres suivants :
a) on utilise un excès allant jusqu'à 20 % de bases fortes,
b) on effectue la réaction de condensation pendant des durées de réaction allant de 1 à 6, de préférence de 1 à 3 heures,
c) on opère avec des concentrations allant de 0,1 à 1,5, de préférence de 0,3 à 0,9 mol/1 d'ester d'acide dicarboxylique à condenser à amener non dilué, par litre de solvant,
d) en évitant la technique habituelle de dilution,
soit on effectue la réaction dans un dispositif clos dans lequel la base dissoute est menée en recirculation et l'ester d'acide dicarboxylique non dilué est introduit par portions dans ce circuit en utilisant une buse d'éjection,
soit le condensat qui se forme, montant, sert en même temps dans une colonne à corps de garnissage pour diluer et pour chauffer l'ester d'acide dicarboxylique amené par fractions non dilué à la tête de la colonne.

2. Procédé de préparation et d'isolement d'héxahydroazépinols ainsi que de leurs sels, par réduction des hexahydroazépinones produites selon la revendication 1, au moyen d'hydrures complexes ou d'hydrogène sur un contact actif hydrogénolytiquement, dans l'eau ou les alcools.

3. Procédé de préparation du chlorhydrate d'azelastine, caractérisé en ce qu'
a) on fait réagir le composé I produit selon la revendication 1 dans lequel :
X signifie CH₂
Y signifie N-CH₃
Z signifie O,
sous forme de son sel d'addition avec un acide avec l'acide chlorhydrique dans un solvant organique avec la benzoylhydrazine et on réduit le composé intermédiaire sans isolation en une benzoylazépinylhydrazine et,
b) on transforme ce composé après coupure acide du reste benzoyle dans l'eau ou dans un solvant organique, avec l'acide 2-(4-chlorophénacétyl)benzoïque, en azélastine et on l'isole sous la forme d'un sel d'addition, avec un acide.

4. Procédé de préparation du chlorhydrate de Flezelastine, caractérisé en ce qu'
a) on fait réagir le composé I produit selon la revendication 1 dans lequel :
X = CH₂
Y = N-CH₂-CH₂C₆H₅
Z = O,
sous forme de son sel d'addition avec un acide dans un solvant organique avec la benzoylhydrazine et on réduit le composé intermédiaire sans isolation en une benzoylazépinylhydrazine,
b) on transforme ce composé après coupure acide du reste benzoyle dans l'eau ou dans des solvants organiques, en Flezélastine avec l'acide 2-(4-fluorophénacétyl)benzoique et on l'isole sous la forme d'un sel d'addition avec un acide.
